(19) Europäisches Patentamt
European Patent Office
Office européen des brevets

(11) EP 0 862 554 B1

(12) **EUROPÄISCHE PATENTSCHRIFT**

(45) Veröffentlichungstag und Bekanntmachung des Hinweises auf die Patenterteilung:
**20.10.2004 Patentblatt 2004/43**

(21) Anmeldenummer: **96934302.9**

(22) Anmeldetag: **05.11.1996**

(51) Int Cl.$^7$: **C07D 213/80**, A61K 31/44

(86) Internationale Anmeldenummer:
**PCT/CH1996/000390**

(87) Internationale Veröffentlichungsnummer:
**WO 1997/018192 (22.05.1997 Gazette 1997/22)**

# (54) AROMATISCHE CARBONSÄUREESTER, IHRE HERSTELLUNG UND VERWENDUNG ALS HEILMITTEL

AROMATIC CARBOXYLIC ACID ESTERS, THE PRODUCTION OF SAME AND USE OF SAME AS MEDICAMENTS

ESTERS CARBOXYLIQUES AROMATIQUES, LEUR PRODUCTION ET LEUR UTILISATION COMME MEDICAMENTS

(84) Benannte Vertragsstaaten:
**AT BE CH DE DK ES FI FR GB GR IE IT LI LU NL PT SE**

(30) Priorität: **16.11.1995 CH 324995**

(43) Veröffentlichungstag der Anmeldung:
**09.09.1998 Patentblatt 1998/37**

(73) Patentinhaber: **F. HOFFMANN-LA ROCHE AG
4070 Basel (CH)**

(72) Erfinder:
- **KLAUS, Michael
D-79576 Weil am Rhein (DE)**
- **MOHR, Peter
CH-4054 Basel (CH)**

(74) Vertreter: **Kjellsaa-Berger, Hanny, Dr. et al
F.Hoffmann-La Roche AG
Patent Department (PLP),
124 Grenzacherstrasse
4070 Basel (CH)**

(56) Entgegenhaltungen:
**EP-A- 0 617 020**
**WO-A-93/06086**
**WO-A-94/15902**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft neue aromatische Carbonsäureester, ihre Herstellung und Verwendung als Heilmittel. Retinoide sind Verbindungen, die je nach Struktur vielfältig eingesetzt werden können, zum Beispiel werden in WO A 93/06086 Nicotinsäure Derivate als selektive Liganden des Retinsäure-γ-Rezeptors beschrieben; und in WO A 94/15902 sind weitere Retinoide als selektive Liganden des Retinsäure-γ-Rezeptors beschrieben. Die Erfindung betrifft insbesondere Ester aromatischer Carbonsäuren mit Hydroxy- oder Mercapto-pyridincarbonsäuren und - säureestern der Formel

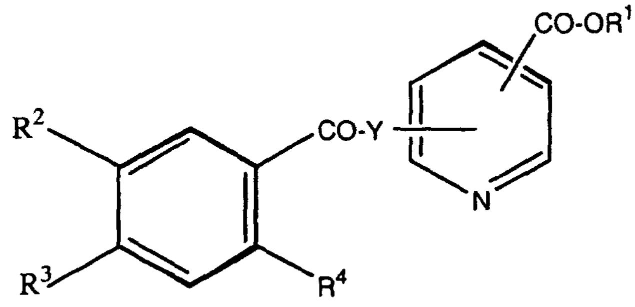

I

worin

| | |
|---|---|
| $R^1$ | Wasserstoff oder $C_{1-6}$-Alkyl; |
| $R^2$ | $C_{1-6}$-Alkyl; |
| $R^3$ | $C_{1-6}$-Alkyl; oder |
| $R^2$ und $R^3$ | zusammen $-(CR^6R^7)_n-$; |
| $R^4$ | $C_{2-8}$-Alkyl, $C_{2-8}$-Alkenyl, $C_{2-8}$-Alkinyl, $-OCH_2R^5$ oder $C_{2-8}$-Alkanoyl; $R^5$ $C_{1-6}$-Alkyl, $C_{2-6}$-Alkenyl oder $C_{2-6}$-Alkinyl; |
| $R^6$ und $R^7$ | Wasserstoff oder $C_{1-6}$-Alkyl; |
| Y | Sauerstoff oder Schwefel; und |
| n | 3, 4 oder 5 |

bedeuten,
und pharmazeutisch anwendbare Salze von Carbonsäuren der Formel I.

Die Erfindung betrifft weiterhin pharmazeutische Präparate auf der Basis von Verbindungen der Formel I oder deren Salzen, Verfahren zur Herstellung von Verbindungen der Formel I und die Verwendung von Verbindungen der Formel I als Heilmittel, sowie als Wirkstoffe bei der Herstellung von Heilmitteln.

Die hier verwendeten Bezeichnungen "$C_{1-6}$", "$C_{2-6}$", und "$C_{2-8}$" stehen für Gruppen mit 1-6, 2-6 und 2-8 Kohlenstoffatomen. Alkylreste können geradkettig oder verzweigt sein. Die Alkylreste $R^1$ sind vorzugsweise geradkettig, wie Methyl, Aethyl, Propyl, Butyl, Pentyl, Hexyl. Alkylreste $R^2$ und $R^3$ sind vorzugsweise verzweigte Alkylreste wie tert. -Butyl. Alkylreste $R^4$ und $R^5$ sind vorzugsweise geradkettig, wie Aethyl, Propyl, Butyl, Pentyl, Hexyl, Heptyl und Octyl. Beispiele von Alkenylresten sind vorzugsweise geradkettige Alkenylreste wie Vinyl, 1- und 2-Propenyl, und 2-Butenyl. Beispiele von Alkinylresten sind Aethinyl, 1- und 2-Propinyl, 1- und 2-Butinyl. Beispiele von $C_{2-8}$-Alkanoylresten sind vorzugsweise geradkettige Alkanoylreste wie Acetyl, Propionyl, Butyryl, Pentanoyl, Hexanoyl, Heptanoyl und Octanoyl.

In einer bevorzugten Ausführungsform der Erfindung ist der Pyridincarbonsäurerest in den Verbindungen der Formel I ein Nicotinsäurerest, insbesondere ein in 6-Stellung verknüpfter Nicotinsäurerest. Weiterhin sind Verbindungen der Formel I bevorzugt, in denen Y Sauerstoff ist, sowie solche, in denen $R^2$ und $R^3$ zusammen $-(CR^6R^7)_n-$ und $R^6$ und $R^7$ Wasserstoff oder Methyl darstellen, insbesondere Verbindungen der Formel I, in denen $R^2$ und $R^3$ zusammen einen Rest $-C(CH_3)_2CH_2CH_2C(CH_3)_2-$, $-C(CH_3)_2(CH_2)_4-$ oder $-C(CH_3)_2(CH_2)_2-$ darstellen. $R^4$ ist vorzugsweise Pentyl, Hexyl, 1-Hexenyl, Hexanoyl oder Butoxy.

Beispiele bevorzugter Verbindungen der Formel I sind

6-(3-Hexyl-5,5,8,8-tetramethyl-5,6,7,8-tetrahydro-naphthalin-2-ylcarbonyloxy)-nicotinsäure,
6-(3-Pentyl-5,5,8,8-tetramethyl-5,6,7,8-tetrahydro-naphthalin-2-ylcarbonyloxy)-nicotinsäure,
6-(3-Hexyl-5,5-dimethyl-6,7,8,9-tetrahydro-5H-benzocyclohepten-2-ylcarbonyloxy)-nicotinsäure,
6-(3-Hex-1-enyl-5,5,8,8-tetramethyl-5,6,7,8-tetrahydro-naphthalin-2-ylcarbonyloxy)-nicotinsäure,
6-(6-Hexyl-3,3-dimethyl-indan-5-yl-carbonyloxy)-nicotinsäure,
6-(3-Butoxy-5,5,8,8-tetramethyl-5,6,7,8-tetrahydro-naphthalin-2-ylcarbonyloxy)-nicotinsäure,

6-(3-Hexanoyl-5,5,8,8-tetramethyl-5,6,7,8-tetrahydro-naphthalin-2-ylcarbonyloxy)-nicotinsäure,
6-(3-Hexyl-5,5,8,8-tetramethyl-5,6,7,8-tetrahydro-naphthalin-2-ylcarbonylsulfanyl)-nicotinsäure.

Die Verbindungen der Formel I können erfindungsgemäss dadurch hergestellt werden, dass man eine Verbindung der Formel II

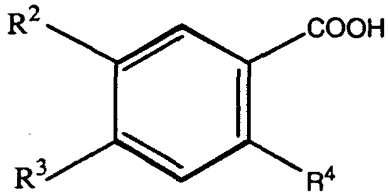

II

worin $R^2$, $R^3$ und $R^4$ die oben angegebene Bedeutung habenmit einer Verbindung der Formel III

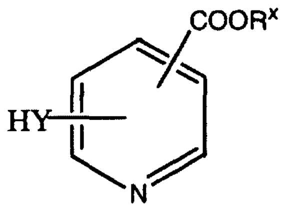

III

worin $R^x$ eine Schutzgruppe darstellt und Y die oben angegebene Bedeutung hat, umsetzt.

Die Veresterung der Verbindung der Formel II mit einer Verbindung der Formel III kann mittels an sich bekannter Methoden bewerkstelligt werden. Zweckmässig erfolgt die Veresterung in Gegenwart eines Kondensationsmittels, wie Dicyclohexylcarbodiimid/Dimethylaminopyridin, in einem geeigneten organischen Lösungsmittel, z.B. einem halogenierten Kohlenwasserstoff wie Methylenchlorid. Beispiele für Schutzgruppen $R^x$ in Verbindungen der Formel III und einer in Verbindungen der Formel II gegebenenfalls anwesenden Hydroxygruppe $R^3$ sind hydrogenolytisch, durch Uebergangsmetallkatalyse (zB. durch $Pd^{(0)}$), oder durch Behandlung mit $F^-$-Ionen abspaltbare Schutzgruppen wie Benzyl, Allyl, Trimethylsilylethyl oder 4-Trimethylsilyl-(E)-but-2-enyl. Beispiele von Oxoschutzgrupen sind insbesondere Ketalgruppen, wie Ethylendioxy. Die Einführung und Abspaltung solcher Schutzgruppen kann in an sich bekannter Weise erfolgen.

Beispiele von Salzen, in die die Carbonsäuren der Formel I übergeführt werden können, sind Alkalimetallsalze, wie Na- und K-Salze, Erdalkalimetallsalze, wie Ca- und Mg-Salze, und Ammoniumsalze, z.B. Salze mit Alkylaminen und Hydroxyalkylaminen oder mit anderen organischen Basen, wie Dimethylamin, Diäthanolamin und Piperidin.

Die als Ausgangsmaterial verwendeten Verbindungen der Formeln II und III können, soweit sie nicht bekannt oder nachstehend beschrieben sind, in Analogie zu bekannten oder den nachstehend beschriebenen Verfahren hergestellt werden.

Die erfindungsgemässen Verbindungen wirken als selektive Liganden des Retinsäure-γ-Rezeptors (RAR-γ). Sie können zur Therapie und Prophylaxe von licht- und altersbedingten Schäden der Haut sowie zur Förderung der Wundheilung, beispielsweise von Schnittwunden, wie Operationswunden, Verbrennungswunden und anderen, von cutanen Traumata herrührenden Wunden, verwendet werden. Die Eignung der erfindungsgemässen Verbindungen für diesen Zweck kann in den in Science 237: 1333-1336 (1987) und J. Pathol. 129: 601-613 (1987) beschriebenen Modellen gezeigt werden. Weiterhin können die erfindungsgemässen Verbindungen zur Therapie und Prophylaxe von dermatologischen Erkrankungen, die mit Epithelläsionen einhergehen, z.B. Akne und Psoriasis, sowie malignen und prämalignen Epithelläsionen, Tumoren und präcancerösen Veränderungen der Schleimhäute in Mund, Zunge, Kehlkopf, Oesophagus, Blase, Cervix und Colon verwendet werden. Die Verbindungen der Formel I zeichnen sich durch geringe hautreizende Wirkung und ein geringes teratogenes Potential aus.

Die Verbindungen der Formel I und deren Salze können dementsprechend in Form pharmazeutischer Präparate Anwendung finden.

Die zur systemischen Anwendung dienenden Präparate können z.B. dadurch hergestellt werden, dass man eine Verbindung der Formel I oder ein Salz davon als wirksamen Bestandteil nichttoxischen, inerten, an sich in solchen Präparaten üblichen festen oder flüssigen Trägern zufügt.

Die Mittel können enteral, parenteral oder topisch verabreicht werden. Für die enterale Applikation eignen sich z.B. Mittel in Form von Tabletten, Kapseln, Dragées, Sirupen, Suspensionen, Lösungen und Suppositorien. Für

die parenterale Applikation sind Mittel in Form von Infusions- oder Injektionslösungen geeignet.

Für die enterale und parenterale Verabreichung können die Verbindungen der Formel I in Mengen von etwa 1-100 mg, vorzugsweise 5-30 mg/Tag, an Erwachsene verabreicht werden.

Zur topischen Anwendung werden die Wirkstoffe zweckmässig in Form von Salben, Tinkturen, Cremen, Lösungen, Lotionen, Sprays, Suspensionen und dergleichen verwendet. Bevorzugt sind Salben und Cremen sowie Lösungen. Diese zur topischen Anwendung bestimmten Präparate können dadurch hergestellt werden, dass man die Verfahrensprodukte als wirksamen Bestandteil nichttoxischen, inerten, für topische Behandlung geeigneten, an sich in solchen Präparaten üblichen festen oder flüssigen Trägern zumischt.

Für die topische Anwendung sind zweckmässig ca. 0,001-1%ige, vorzugsweise 0,3-2%ige Lösungen, sowie ca. 0,001-1%ige, vorzugsweise 0,01-1%ige, Salben oder Cremen geeignet.

Den Präparaten kann gegebenenfalls ein Antioxydationsmittel, z.B. Tocopherol, N-Methyl-g-tocopheramin sowie butyliertes Hydroxyanisol oder butyliertes Hydroxytoluol beigemischt werden.

Die Wirksamkeit der erfindungsgemässen Verbindungen bei der Behandlung lichtgeschädigter Haut kann mittels der nachstehend beschriebenen Versuchsanordnungen beurteilt werden:

<u>Behebung UV-B-verursachter Hautschäden bei der haarlosen Maus</u>

Haarlose Mäuse (HRS/J Stamm, Jackson Labs, zu Beginn des Experiments 5-7 Wochen alt) wurden dreimal wöchentlich mit einer Anordnung von 8 Westinghouse Bestrahlungslampen (FS40), die etwa 20 cm oberhalb der Tiere plaziert waren, bestrahlt. Die Strahlungsdosis wurde durch ein handelsübliches, phototherapeutisches Kontrollgerät gesteuert. Die UV-B Dosisleistung wurde so gewählt, dass sie selten 0,06J/cm$^2$/h überschritt und ein minimales Erythem, jedoch keine Verbrennungen oder Narben verursachte. Nach einer Gesamtdosis von etwa 3,5J/cm$^2$ (nach 5-6 Monaten) ergab sich eine durch histologischen Befund gesicherte signifikante Elastosis, die durch Messungen des Elastins mittels Radioimmunoassay für Desmosin in der gesamten Haut bestätigt wurde. Der Desmosingehalt erhöhte sich um das zwei- bis dreifache nach 3,5J/cm$^2$ UV-B Bestrahlung. Um die Hautschäden zu beheben, wurde die UV-Bestrahlung unterbrochen und Gruppen der Tiere wurden separat dreimal wöchentlich mit verschiedenen Dosen von Verbindungen der Formel I, in Aceton gelöst, behandelt. Die Lösungen wurden jede Woche frisch so hergestellt, dass die zu verabreichende Dosis in 100 ml Aceton enthalten war, und topisch auf ein Gebiet von etwa 10 cm$^2$ auf den Rücken der Tiere aufgebracht. Eine Kontrollgruppe wurde nur mit Aceton behandelt.

Nach 10 Behandlungswochen wurden die Tiere getötet, Hautpräparate angefertigt und durch Luna-Färbung des Elastins und Van Gieson-Färbung des Collagens das Ausmass der Wiederherstellung quantitativ gemessen. Auf dieser Basis wurde eine Aktivitätsskala aufgestellt mit einer Bewertung von 0 (inaktiv) bis 4 (vollständige, zusammenhängende Wiederherstellung). All-trans-Retinsäure und 13-cis-Retinsäure zeigten in diesem Versuchsmodell eine Aktivität von 2, die geprüften Verbindungen der Formel I dagegen eine Aktivität von 4.

Die Erfindung wird durch die nachstehenden Beispiele weiter erläutert:

<u>Beispiel 1</u>

6 g 3-Hexyl-5,5,8,8-tetramethyl-5,6,7,8-tetrahydro-naphthalin-2-ylcarbonsäure wurden in 180 ml Methylenchlorid gelöst. Nach Zugabe einer Lösung von 4,8 g 6-Hydroxy-nicotinsäurebenzylester in 160 ml Methylenchlorid und von 2,3 g 4-Dimethylaminopyridin wurde die Lösung auf 0°C abgekühlt und mit 4,4 g Dicyclohexylcarbodiimid versetzt. Das Reaktionsgemisch wurde 1 Stunde bei 0°C und 2 Stunden bei Raumtemperatur gerührt, danach auf eiskalte wässrige Ammoniumchlorid-Lösung gegossen, mehrfach mit Aether extrahiert, mit Wasser gewaschen, über Natriumsulfat getrocknet und eingedampft. Das mehrheitlich kristalline Rohprodukt wurde über Kieselgel filtriert (Eluierungsmittel Hexan/Essigester = 9:1) und aus Hexan umkristallisiert. Man erhielt nach erneuter Kristallisation der Mutterlauge zusammen 5,7 g 6-(3-Hexyl-5,5,8,8-tetramethyl-5,6,7,8-tetrahydro-naphthalin-2-yl-carbonyloxy)-nicotinsäurebenzylester in farblosen Kristallen, Smp. 94-95°C.

Dieser Benzylester (5,7 g) wurde in 450 ml Essigester gelöst und nach Zugabe von 2,2 g 10% Palladiumkohle mit Wasserstoff unter Normaldruck bei Raumtemperatur hydriert. Nach 40 Minuten wurde die Hydrierung abgebrochen, vom Katalysator abfiltriert, eingedampft und der schwach bräunliche Rückstand aus Essigester/Hexan umkristallisiert. Man erhielt 2,8 g 6-(3-Hexyl-5,5,8,8-tetramethyl-5,6,7,8-tetrahydro-naphthalin-2-ylcarbonyloxy)-nicotinsäure in farblosen Kristallen, Smp. 146-149°C (Zers.).

Die als Ausgangsprodukt verwendete Carbonsäure wurde wie folgt hergestellt:

15 g 6-Brom-7-hexyl-1,1,4,4-tetramethyl-1,2,3,4-tetrahydro-naphthalin wurden in 180 ml Tetrahydrofuran gelöst und bei -78°C tropfenweise mit 64,5 ml einer 1,5 molaren Lösung von tert.Butyl-lithium in Pentan versetzt. Nach 1-stündigem Rühren bei -78°C wurde während 2 Stunden ein kräftiger Strom von Kohlendioxid in das Reaktionsgefäss eingeleitet. Anschliessend wurde das Reaktionsgemisch durch Zugabe von 190 ml 2N Salzsäure angesäuert, mehrfach mit Essigester extrahiert, mit Wasser gewaschen, über Natriumsulfat getrocknet und eingedampft. Das ölige Rohprodukt wurde über Kieselgel filtriert (Eluierungsmittel Hexan, dann Hexan/Essigester = 4:1) und aus Hexan umkristallisiert. Man erhielt 7,6 g 3-Hexyl-5,5,8,8-tetramethyl-5,6,7,8-tetrahydro-naphthalin-2-yl-carbonsäure in farblosen Kristallen, Smp. 98-99°C.

Der als Ausgangsprodukt verwendete 6-Hydroxy-nicotinsäurebenzylester wurde wie folgt hergestellt:

30 g 6-Hydroxynicotinsäure wurden mit 500 ml Benzylalkohol und 1 ml konzentrierter Schwefelsäure versetzt. Nach 3-tägigem Kochen am Rückfluss wurde das Reaktionsgemisch auf Eiswasser gegossen, mehrfach mit Essigester extrahiert, mit Wasser gewaschen, über Natriumsulfat getrocknet und eingedampft, zunächst am Wasserstrahlvakuum, dann am Hochvakuum. Der halbkristalline Rückstand wurde über Kieselgel filtriert (Eluierungsmittel Hexan/Essigester = 2:1, dann Essigester) und aus Essigester/Hexan umkristallisiert. Man erhielt 19 g 6-Hydroxy-nicotinsäurebenzylester in farblosen Kristallen, Smp. 178-179°C.

Beispiel 2

980 mg 3-Hexyl-5,5-dimethyl-6,7,8,9-tetrahydro-5H-benzocyclohepten-2-carbonsäure wurden in 5 ml $CH_2Cl_2$ vorgelegt und mit 745 mg 6-Hydroxynicotinsäeure benzyl ester und 32 mg 4-Dimethylaminopyridin versetzt. Bei 0° gab man 738 mg (1Eq.) Dicyclohexylcarbodiimid dazu und liess 1 h bei Raumtemperatur reagieren. Man verdünnte mit Diethylether, adsorbierte an Merck Kieselgel 60, dampfte ein und chromatographierte den Rückstand an $SiO_2$ (Hexan/ Ethylacetat=93/7). Dabei isolierte man 1.19 g 6-(3-Hexyl-5,5-dimethyl-6,7,8,9-tetrahydro-5H-benzocyclohepten-2-yl-carbonyloxy)-nicotinsäeure benzylester als farbloses Oel.

1.37 g 6-(3-Hexyl-5,5-dimethyl-6,7,8,9-tetrahydro-5H-benzocyclohepten-2-yl-carbonyloxy)-nicotinsäure benzyl ester wurden in 26 ml Ethylacetat vorgelegt und über 137 mg Pd/C (5%) bei Raumtemperatur und 1 Atm. $H_2$ während 4 h hydriert. Man filtrierte über Cellit, dampfte zur Trockne ein und kristallisierte aus Hexan/Ethylacetat um. Dabei resultierten 900 mg 6-(3-Hexyl-5,5-dimethyl-6,7,8,9-tetrahydro-5H-benzocyclohepten-2-ylcarbonyloxy)-nicotinsäeure als farblose Kristalle vom Smp. 119-120°.

Herstellung des Ausgangsmaterials:

> 3.58 g 2-Brom-9,9-dimethyl-6,7,8,9-tetrahydro-5H-benzocyclohepten (Herstellung beschrieben in Eur. Pat. Anmeldung EP 410,358 A) wurden unter Ar in 10 ml Piperidin vorgelegt. Man gab nacheinander 160 mg $((Ph)_3P)_4Pd$, 47 mg CuI und 45 mg $(Ph)_3P$ zu und tropfte dann bei 80° 4.8 ml (3Eq.) 1-Hexin innert 1 h zu. Man liess 2 h bei gleicher Temperatur nachreagieren, goss dann auf Eis/HCl, extrahierte mit Hexan, wusch zweimal mit $H_2O$, trocknete über $Na_2SO_4$ und dampfte zur Trockne ein. Flash-Chromatographie an $SiO_2$ (Hexan) ergab 3.25 g 2-Hex-1-ynyl-9,9-dimethyl-6,7,8,9-tetrahydro-5H-benzocyclohepten als farbloses Oel (GC-Reinheit:98%).

Dieses Alkin wurde in 30 ml Ethylacetat gelöst und über 2 g Pd/C (10%) bei Raumtemperatur und 1 Atm $H_2$ hydriert. Filtration über Cellit und Entfernen des Lösungsmittels ergab 3.08 g 2-Hexyl-9,9-dimethyl-6,7,8,9-tetrahydro-5H-benzocyclohepten als farbloses Oel (GC-Reinheit:98%).

Diese 3.08 g 2-Hexyl-9,9-dimethyl-6,7,8,9-tetrahydro-5H-benzocyclohepten wurden in 35 ml $CH_2Cl_2$ gelöst, bei 0° mit einer Spatelspitze Fe-Pulver versetzt und dann mit einer Lösung von 0.67 ml $Br_2$ (1.1 Eq.) in 5 ml $CH_2Cl_2$ zur Reaktion gebracht. Nach 30 Min. goss man auf Eis, extrahierte mit Diethylether, wusch mit Bisulfit-Lauge und NaCl-Lsg., trocknete über $Na_2SO_4$ und dampfte zur Trockne ein. Flash-Chromatographie an $SiO_2$ (Hexan) ergab 3.21 g 2-Brom-3-hexyl-5,5-dimethyl-6,7,8,9-tetrahydro-5Hbenzocyclohepten als farbloses Oel (GC-Reinheit: 82%, daneben 2% Ed., 2.6% Regioisomeres, 8.3% Dibromverbindung und weitere Prod.)

Diese 3.21 g 2-Brom-3-hexyl-5,5-dimethyl-6,7,8,9-tetrahydro-5Hbenzocyclohepten wurden unter Ar in 30 ml abs. Tetrahydrofuran vorgelegt und bei -78° mit 6.75 ml 1.55 M nBuLi (Hexan) (1.1 Eq.) versetzt. Der Metall/Halogen-Austausch wurde mittels GC verfolgt. Nach 40 Min. leitete man einen grossen Ueberschuss $CO_2$-Gas ein und entfernte das Kühlbad. Nach 10 Min. goss man auf Eis/$NH_4Cl$-Lösung, extrahierte mit Diethylether, wusch mit $H_2O$, trocknete über $Na_2SO_4$ und entfernte die Lösungsmittel i.V. Flash-Chromatographie an $SiO_2$ (Hexan/Ethylacetat=8/2) lieferte 2.26 g 3-Hexyl-5,5-dimethyl-6,7,8,9-tetrahydro-5H-benzocyclohepten-2-carbonsäure als weisse Kristalle vom Smp. 74-75°.

Beispiel 3

In Analogie zu Beispiel 2 wurde, ausgehend von 5-Brom-1,1-dimethylindan (Herstellung beschrieben in Org. Prep. Proced. Int. *10*, 123 (1978)) die 6-(6-Hexyl-3,3-dimethyl-indan-5-yl-carbonyloxy)-nicotinsäure als weisse Kristalle vom Smp. 104-105°C erhalten.

Beispiel 4

805 mg (Z)-3-Hex-1-enyl-5,5,8,8-tetramethyl-5,6,7,8-tetrahydronaphthalin-2-carbonsäure wurden in 8 ml $CH_2Cl_2$ gelöst und mit 679 mg (1 Eq.) 6-Hydroxy-nicotinsäure 4-trimethylsilanyl-(E)-but-2-enyl ester und 24 mg 4-Dimethylaminopyridin versetzt. Bei 0° gab man 581 mg (1 Eq.) Dicyclohexylcarbodiimid zu und liess 4 h bei Raumtemperatur reagieren.Man filtrierte vom ausgefallenen Harnstoff ab, dampfte ein und reinigte das Rohprodukt mittels Flash-Chromatographie an $SiO_2$ (Hexan/Aethylacetat= 95/5). Dabei isolierte man 971 mg 6-(3-(Z)-1-Hexenyl-5,5,8,8-tetramethyl-5,6,7,8-tetrahydro-naphthalin-2-yl-carbonyloxy)-nicotinsäure 4-trimethylsilanyl-(E)-but-2-enyl ester als farblosen Lack.

970 mg 6-(3-(Z)-1-Hexenyl-5,5,8,8-tetramethyl-5,6,7,8-tetrahydronaphthalin-2-yl-carbonyloxy)-nicotinsäure 4-trimethylsilanyl-(E)-but-2-enyl ester wurden in 8 ml $CH_2Cl_2$ gelöst und bei Raumtemperatur mit 40 mg (2 Mol %) $((Ph)_3P)_4Pd$ versetzt. Man rührte während 4 1/2 h unter Sauerstoffausschluss, goss dann auf 100 ml Aethylacetat, in dem 4.5 g $SiO_2$ aufgeschlämmt waren, und rührte 10 Min. Man nutschte ab, wusch gründlich mit Aethylacetat nach und dampfte das Filtrat zur Trockne ein. Dreimaliges Umkristallisieren aus Hexan/Aethylacetat ergab schliesslich 334 mg 6-(3-(Z)-1-Hexenyl-5,5,8,8-tetramethyl-5,6,7,8-tetrahydro-naphthalin-2-yl-carbonyloxy)-nicotinsäure als weisse Kristalle vom Smp. 133-35°(dec.).

Der 6-Hydroxy-nicotinsäure 4-trimethylsilanyl-(E)-but-2-enyl ester wurde wie folgt hergestellt:

In 30 ml abs. Dimethylformamid legte man nacheinander vor: 1.3 g 4-Trimethylsilyl-(E)-but-2-en-1-ol (Synthese beschrieben in *J. Org. Chem.* **1984,** 49, 4092), 1.08 g 6-Hydroxy-nicotinsäure und 178 mg 4-Dimethylaminopyridin. Bei 0° gab man 1.77 g (1.1 Eq.) Dicyclohexylcarbodiimid zu und liess über Nacht bei Raumtemperatur nachreagieren. Man filtrierte vom ausgefallenen Harnstoff ab, extrahierte mit Aethylacetat, wusch zweimal mit $H_2O$, trocknete über $Na_2SO_4$ und entfernte das Lösungsmittel unter vermindertem Druck. Flash-Chromatographie an $SiO_2$ (Hexan/ Aethylacetat=1/1) lieferte 782 mg 6-Hydroxy- nicotinsäure 4-trimethylsilanyl-(E)-but-2-enyl ester als weisse Kristalle vom Smp. 107-110°( dec.).

Die (Z)-3-Hex-1-enyl-5,5,8,8-tetramethyl-5,6,7,8-tetrahydro-naphthalin-2-yl-carbonsäure wurde wie folgt hergestellt:

15 g 5,5,8,8-Tetramethyl-tetrahydro-naphthalin wurden in 160 ml $CH_2Cl_2$ gelöst, mit einer Spatelspitze Fe-Pulver versetzt und dann mit einer Lösung von 10.1 ml $Br_2$ (2.5 Eq.) in 50 ml $CH_2Cl_2$ umgesetzt. Nach 1/4 h goss man auf Eis, extrahierte mit Diäthyläther, wusch mit Bisulfit-Lauge und $H_2O$, trocknete über $Na_2SO_4$ und dampfte zur Trockne ein. Filtrier-Säule über $SiO_2$ (Hexan) ergab 25.3 g 2,3-Dibrom-5,5,8,8-tetramethyl-tetrahydronaphthalin als gelbliche Kristalle vom Smp. 117-120°.

10 g 2,3-Dibrom-5,5,8,8-tetramethyl-5,6,7,8-tetrahydro-naphthalin wurden in 25 ml Piperidin vorgelegt. Man gab nacheinander 668 mg $((Ph)_3P)_4Pd$, 211 mg CuI und 291 mg $(Ph)_3P$ zu und tropfte dann bei 75-80° 5.16 ml (1.59 Eq.) 1-Hexin, gelöst in 12 ml Piperidin, innert 2 1/2 h zu. Man liess 1 h bei gleicher Temperatur nachreagieren, goss dann auf Eis/HCl, extrahierte mit Diäthyläther, wusch mit $H_2O$, trocknete über $Na_2SO_4$ und dampfte zur Trockne ein. Flash-Chromatographie an $SiO_2$ (Hexan) ergab 6.67 g 6-Brom-7-hex-1-ynyl-1,1,4,4-tetramethyl-1,2,3,4-tetrahydronaphthalin als gelbliche Kristalle vom Smp. 59-63°.

6.67 g 6-Brom-7-hex-1-ynyl-1,1,4,4-tetramethyl-1,2,3,4-tetrahydronaphthalin wurden in 270 ml Aethanol gelöst und bei 40° und 1 Atm $H_2$ während mehrerer Tage in Gegenwart von Lindlar-Katalysator hydriert. Die Reaktion verlief äusserst harzig. Nach 0h, 24h, 48h, usw. wurden jeweils 6.7 g frischer Katalysator zugegeben; nach 5 Tagen wurde über Cellit abfiltriert und neu gestartet. Nach insgesamt 9 Tagen wurde erneut vom Katalysator abfiltriert, das Lösungsmittel unter vermindertem Druck entfernt und der Rückstand mittels Flash-Chromatographie an $SiO_2$ (Hexan) gereinigt. Man erhielt 3.72 g Produkt, das nach GC 75% (Z)-6-Brom-7-hex-1-enyl-1,1,4,4-tetramethyl-1,2,3,4-tetrahydro-naphthalin enthielt.

3.72 g (Z)-6-Brom-7-hex-1-enyl-1,1,4,4-tetramethyl-1,2,3,4-tetrahydronaphthalin (75% rein) wurden in 25 ml abs. Tetrahydrofuran vorgelegt und bei -75° mit 8.25 ml 1.55 M nBuLi (Hexan) (1.2 Eq.) versetzt. Nach 1/2 h leitete man während 30 Min. einen kräftigen $CO_2$-Strom über die Lösung, wobei trotz unverändeter Kühlung die Temperatur auf -60° stieg. Man liess auf Raumtemperatur erwärmen, goss auf Eis/HCl-Lösung, extrahierte mit Diäthyläther, wusch mit $H_2O$, trocknete über $Na_2SO_4$ und entfernte die Lösungsmittel i.V. Zweimalige Flash-Chromatographie an $SiO_2$ (Hexan/Aethylacetat=85/15, dann 9/1) lieferte 1.06g (Z)-3-Hex-1-enyl-5,5,8,8-tetramethyl-5,6,7,8-tetrahydro-naphthalin-2-carbonsäure als farblose Kristalle.

Beispiel 5

In Analogie zu Beispiel 1 wurde durch Reaktion von 6-Brom-1,1,4,4-tetramethyl-7-pentyl-1,2,3,4-tetrahydronaphthalin mit tert.Butyl-Lithium und anschliessendem Einleiten von Kohlendioxid-Gas 5,5,8,8-Tetramethyl-3-pentyl-5,6,7,8-tetrahydro-naphthalin-2-carbonsäure (Smp. 123-124°C, aus Hexan) hergestellt. Umsetzung dieser Säure mit 6-Hydroxy-nicotinsäurebenzylester ergab nach Umkristallisation aus Hexan 6-(3-Pentyl-5,5,8,8-tetramethyl-5,6,7,8-tetrahydro-naphthalin-2-yl-carbonyloxy)-nicotinsäurebenzylester, Smp. 94-95°C.

Entfernung der Benzylschutzgruppe durch Hydrierung mit Wasserstoff/Palladiumkohle lieferte nach Umkristallisation aus Essigester/Hexan das gewünschte Endprodukt 6-(3-Pentyl-5,5,8,8-tetramethyl-5,6,7,8-tetrahydronaphthalin-2-yl-carbonyloxy)-nicotinsäure in weissen Kristallen, Smp. 139-141°C.

Beispiel 6

In Analogie zu Beispiel 1 wurde durch Metallierung von 6-Brom-7-pentoxy-1,1,4,4-tetramethyl-1,2,3,4-tetrahydro-naphthalin mit tert.Butyl-Lithium und Umsetzung mit Kohlendioxid-Gas 3-Pentoxy-5,5,8,8-tetramethyl-5,6,7,8-tetrahydro-naphthalin-2-carbonsäure (Smp. 67-68°C, aus Hexan) hergestellt. Reaktion dieser Säure mit 6-Hydroxy-nicotinsäurebenzylester ergab 6-(3-Pentoxy-5,5,8,8-tetramethyl-5,6,7,8-tetrahydro-naphthalin-2-carbonyloxy)-nicotinsäurebenzylester als farbloses Oel. Anschliessende Hydrierung lieferte das gewünschte Endprodukt 6-(3-Pentoxy-5,5,8,8-tetramethyl-5,6,7,8-tetrahydro-naphthalin-2-yl-carbonyloxy)-nicotinsäure in weissen Kristallen, Smp. 132-133°C (aus Diethylether/ Hexan).

Beispiel A

Hartgelatinekapseln können wie folgt hergestellt werden:

| Bestandteile | mg/Kapsel |
|---|---|
| 1. sprühgetrocknetes Pulver enthaltend 75% Verbindung I | 20 |
| 2. Natriumdioctylsulfosuccinat | 0,2 |
| 3. Natriumcarboxymethylcellulose | 4,8 |
| 4. mikrokristalline Cellulose | 86,0 |
| 5. Talk | 8,0 |
| 6. Magnesiumstearat | 1.0 |
| Total | 120 |

Das sprühgetrocknete Pulver, das auf dem Wirkstoff, Gelatine und mikrokristalliner Cellulose basiert und eine mittlere Korngrösse des Wirkstoffes von <1 μ aufweist (mittels Autokorrelationsspektroskopie gemessen), wird mit einer wässrigen Lösung von Natriumcarboxymethylcellulose und Natriumdioctylsulfosuccinat befeuchtet und geknetet. Die resultierende Masse wird granuliert, getrocknet und gesiebt, und das erhaltene Granulat mit mikrokristalliner Cellulose, Talk und Magnesiumstearat vermischt. Das Pulver wird in Kapseln der Grösse 0 abgefüllt.

Beispiel B

Tabletten können wie folgt hergestellt werden:

| Bestandteile | mg/Tablette |
|---|---|
| 1. Verbindung I als feingemahlenes Pulver | 20 |
| 2. Milchzucker pulv. | 100 |
| 3. Maisstärke weiss | 60 |
| 4. Povidone K30 | 8 |
| 5. Maisstärke weiss | 112 |
| 6. Talk | 16 |
| 7. Magnesiumstearat | 4 |
| Total | 320 |

Die feingemahlene Substanz wird mit Milchzucker und einem Teil der Maisstärke gemischt. Die Mischung wird mit einer wässrigen Lösung von Povidone K30 befeuchtet und geknetet, und die resultierende Masse granuliert, getrocknet und gesiebt. Das Granulat wird mit der restlichen Maisstärke, Talk und Magnesiumstearat vermischt und zu Tabletten geeigneter Grösse verpresst.

Beispiel C

Weichgelatinekapseln können wie folgt hergestellt werden:

| Bestandteile | mg/Kapsel |
|---|---|
| 1. Verbindung I | 5 |
| 2. Triglycerid | 450 |
| Total | 455 |

1 g Verbindung I werden unter Rühren, Inertbegasung und Lichtschutz in 90 g mittelkettigem Triglycerid gelöst. Diese Lösung wird als Kapselfüllmasse zu Weichgelatinekapseln à 5 mg Wirkstoff verarbeitet.

Beispiel D

Eine Lotion kann wie folgt hergestellt werden:

| Bestandteile | |
|---|---|
| 1. Verbindung I, feingemahlen | 1,0 g |
| 2. Carbopol 934 | 0,6 g |
| 3. Natriumhydroxid | q.s. ad pH 6 |
| 4. Aethanol, 94% | 50,0 g |
| 5. entmineralisiertes Wasser | ad 100,0 g |

Der Wirkstoff wird unter Lichtschutz in die Mischung Aethanol, 94%ig/Wasser eingearbeitet. Carbopol 934 wird bis zur vollständigen Gelierung eingerührt und der pH-Wert mit Natriumhydroxid eingestellt.

Beispiel E

Eine Crème kann aus den nachstehend aufgeführten Inhaltsstoffen in an sich bekannter Weise hergestellt werden:

| | Gew.-% |
|---|---|
| Verbindung der Formel I | 0,1-5 |
| Cetylalkohol | 5,25-8,75 |
| Arlacel 165 (Glyceryl/PEG 100-stearat) | 3,75-6,25 |
| Miglyol 818 (Capryl-/Caprin-/Linolsäure triglycerid) | 11,25-18,75 |
| Sorbit-Lösung | 3,75-6,25 |
| EDTA-$Na_2$ | 0,075-0,125 |
| Carbopol 934P (Carbomer 934P) | 0,15-0,25 |
| butyliertes Hydroxyanisol | 0,0375-0,0625 |
| Methylparaben | 0,135-0,225 |
| Propylparaben | 0,0375-0,0625 |
| NaOH (10% solution) | 0,15-0,25 |
| Wasser q.s. | ad 100,00 |

Beispiel F

Ein Gel kann aus den nachstehend aufgeführten Inhaltsstoffen in an sich bekannter Weise hergestellt werden:

| | Gew.-% |
|---|---|
| Verbindung der Formel I | 0,1-5 |
| Pluronic L 101 (Poloxamer 331) | 10,00 |

(fortgesetzt)

| | Gew.-% |
|---|---|
| Aerosil 200 (Siliciumdioxid) | 8,00 |
| PCL Liquid (Fettsäureester) | 15,00 |
| Cetiol V (Decyloleat) | 20,00 |
| Neobee Oil (Triglycerid mittlerer Kettenlänge) | 15,00 |
| Euhanol G (Octyldodecanol), q.s. | ad 100,00 |

Durch Variation der Verhältnisse zwischen den Hilfsstoffen der Beispiele E und F können die physikalischen Eigenschaften der Präparate verändert werden.

## Patentansprüche

1. Verbindungen der Formel

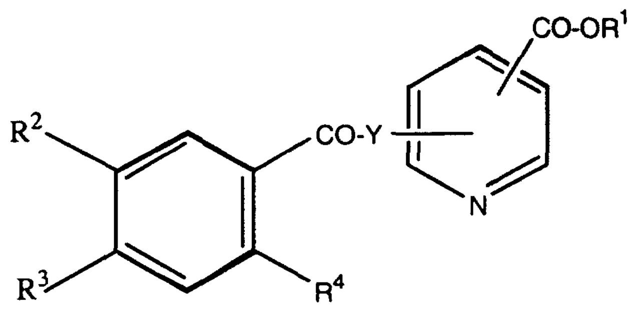

I

worin

| | |
|---|---|
| $R^1$ | Wasserstoff oder $C_{1-6}$-Alkyl; |
| $R^2$ | $C_{1-6}$-Alkyl; |
| $R^3$ | $C_{1-6}$-Alkyl; oder |
| $R^2$ und $R^3$ | zusammen $-(CR^6R^7)_n-$; |
| $R^4$ | $C_{2-8}$-Alkyl, $C_{2-8}$-Alkenyl, $C_{2-8}$-Alkinyl, $-OCH_2R^5$ oder $C_{2-8}$-Alkanoyl; |
| $R^5$ | $C_{1-6}$-Alkyl, $C_{2-6}$-Alkenyl oder $C_{2-6}$-Alkinyl; |
| $R^6$ und $R^7$ | unabhängig voneinander Wasserstoff oder $C_{1-6}$-Alkyl; |
| Y | Sauerstoff oder Schwefel; und |
| n | 3, 4 oder 5 |

bedeuten,
und pharmazeutisch anwendbare Salze von Carbonsäuren der Formel I.

2. Verbindungen gemäss Anspruch 1, worin Y Sauerstoff ist.

3. Verbindungen gemäss Anspruch 1, worin Y Schwefel ist

4. Verbindungen gemäss einem der Ansprüche 1-3, worin $R^2$ und $R^3$ zusammen $-(CR^6R^7)_n-$ bedeuten.

5. Verbindungen gemäss Anspruch 4, worin $R^2$ und $R^3$ zusammen einen Rest $-C(CH_3)_2CH_2CH_2C(CH_3)_2-$, $-C(CH_3)_2(CH_2)_4-$ oder $-C(CH_3)_2(CH_2)_2$bedeuten.

6. Verbindungen gemäss Anspruch 5, worin $R^4$ $C_{2-8}$-Alkyl ist

7. Verbindungen gemäss Anspruch 5, worin $R^4$ $C_{2-8}$-Alkenyl ist.

**8.** Verbindungen gemäss Anspruch 5, worin $R^4$ $C_{2-8}$-Alkanoyl ist.

**9.** Verbindungen gemäss Anspruch 5, worin $R^4$ $-OCH_2R^5$ ist.

**10.** Die Verbindung gemäss Anspruch 6, 6-(3-Hexyl-5,5,8,8-tetramethyl-5,6,7,8-tetrahydro-naphthalin-2-yl-carbonyloxy)-nicotinsäure und pharmazeutisch anwendbare Salze davon.

**11.** Die Verbindungen gemäss Anspruch 6,
6-(3-Pentyl-5,5,8,8-tetramethyl-5,6,7,8-tetrahydro-naphthalin-2-ylcarbonyloxy)-nicotinsäure,
6-(3-Hexyl-5,5-dimethyl-6,7,8,9-tetrahydro-5H-benzocyclohepten-2-ylcarbonyloxy)-nicotinsäure, und
6-(6-Hexyl-3,3-dimethyl-indan-5-yl-carbonyloxy)-nicotinsäure,
und pharmazeutisch anwendbare Salze davon.

**12.** Die Verbindungen gemäss Anspruch 7, 6-(3-(Z)-1-Hexenyl-5,5,8,8-tetramethyl-5,6,7,8-tetrahydro-naphthalin-2-ylcarbonyloxy)-nicotinsäure und pharmazeutisch anwendbare Salze davon.

**13.** Die Verbindungen gemäss Anspruch 9,
6-(3-Butoxy-5,5,8,8-tetramethyl-5,6,7,8-tetrahydro-naphthalin-2-ylcarbonyloxy)-nicotinsäure und
6-(3-Pentoxy-5,5,8,8-tetramethyl-5,6,7,8-tetrahydro-naphthalin-2-ylcarbonyloxy)-nicotinsäure
und pharmazeutisch anwendbare Salze davon.

**14.** Die Verbindungen der Ansprüche 1-13 zur Anwendung als Heilmittel.

**15.** Pharmazeutische Präparate, enthaltend eine Verbindung der Ansprüche 1-13 und übliche pharmazeutische Träger- und Hilfsstoffe.

**16.** Verwendung einer Verbindung der Ansprüche 1-13 als Wirkstoff bei der Herstellung von Heilmitteln zur Therapie und Prophylaxe von licht- und altersbedingten Schäden der Haut; zur Förderung der Wundheilung, zur Therapie und Prophylaxe von dermatologischen Erkrankungen, die mit Epithelläsionen einhergehen; sowie malignen und prämalignen Epithelläsionen, Tumoren und präcancerösen Veränderungen der Schleimhäute in Mund, Zunge, Kehlkopf, Oesophagus, Blase, Cervix und Colon.

**17.** Verfahren zur Herstellung von Verbindungen der Ansprüche 1-13, **dadurch gekennzeichnet, dass** man eine Verbindung der Formel II

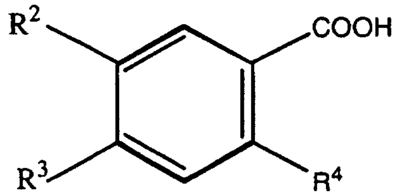

II

worin $R^2$, $R^3$ und $R^4$ die in Anspruch 1 angegebene Bedeutung haben,
mit einer Verbindung der Formel III

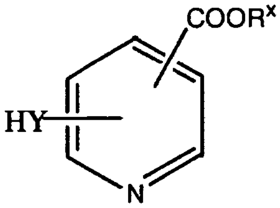

III

worin $R^x$ eine Schutzgruppe darstellt und Y die in Anspruch 1 angegebene Bedeutung hat,
umsetzt.

## Claims

**1.** Compounds of the formula

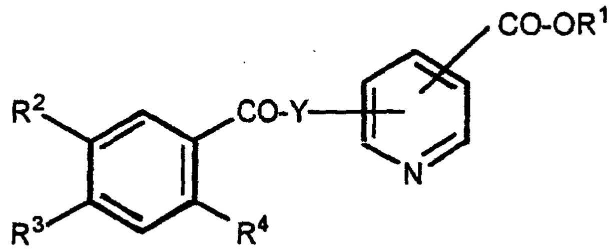

I

wherein

| | |
|---|---|
| $R^1$ | signifies hydrogen or $C_{1-6}$-alkyl; |
| $R^2$ | signifies $C_{1-6}$-alkyl; |
| $R^3$ | signifies $C_{1-6}$-alkyl; or |
| $R^2$ and $R^3$ | together signify $-(CR^6R^7)_n-$; |
| $R^4$ | signifies $C_{2-8}$-alkyl, $C_{2-8}$-alkenyl, $C_{2-8}$-alkynyl, $-OCH_2R^5$ or $C_{2-8}$-alkanoyl; |
| $R^5$ | signifies $C_{1-6}$-alkyl, $C_{2-6}$-alkenyl or $C_{2-6}$-alkynyl; |
| $R^6$ and $R^7$ | independently of each other signify hydrogen or $C_{1-6}$-alkyl; |
| Y | signifies oxygen or sulphur; and |
| n | signifies 3, 4 or 5, |

and pharmaceutically usable salts of carboxylic acids of formula I.

**2.** Compounds in accordance with claim 1, wherein Y is oxygen.

**3.** Compounds in accordance with claim 1, wherein Y is sulphur.

**4.** Compounds in accordance with any one of claims 1-3, wherein $R^2$ and $R^3$ together signify $-(CR^6R^7)_n-$.

**5.** Compounds in accordance with claim 4, wherein $R^2$ and $R^3$ together signify a residue $-C(CH_3)_2CH_2CH_2C(CH_3)_2-$, $-C(CH_3)_2(CH_2)_4-$ or $-C(CH_3)_2(CH_2)_2-$.

**6.** Compounds in accordance with claim 5, wherein $R^4$ is $C_{2-8}$-alkyl.

**7.** Compounds in accordance with claim 5, wherein $R^4$ is $C_{2-8}$-alkenyl.

**8.** Compounds in accordance with claim 5, wherein $R^4$ is $C_{2-8}$-alkanoyl.

**9.** Compounds in accordance with claim 5, wherein $R^4$ is $-OCH_2R^5$.

**10.** The compound in accordance with claim 6,6-(3-hexyl-5,5,8,8-tetramethyl-5,6,7,8-tetrahydro-naphthalen-2-yl-carbonyloxy)-nicotinic acid and pharmaceutically usable salts thereof.

**11.** The compounds in accordance with claim 6,

6-(3-pentyl-5,5,8,8-tetramethyl-5,6,7,8-tetrahydro-naphthalen-2-yl-carbonyloxy)-nicotinic acid,
6-(3-hexyl-5,5-dimethyl-6,7,8,9-tetrahydro-5H-benzocyclohepten-2-yl-carbonyloxy)-nicotinic acid, and
6-(6-hexyl-3,3-dimethyl-indan-5-yl-carbonyloxy)-nicotinic acid,

and pharmaceutically usable salts thereof.

**12.** The compounds in accordance with claim 7,6-(3-(Z)-1-hexenyl-5,5,8,8-tetramethyl-5,6,7,8-tetrahydro-naphthalen-2-ylcarbonyloxy)-nicotinic acid and pharmaceutically usable salts thereof.

**13.** The compounds in accordance with claim 9,
6-(3-butoxy-5,5,8,8-tetramethyl-5,6,7,8-tetrahydro-naphthalen-2-yl-carbonyloxy)-nicotinic acid and
6-(3-pentoxy-5,5,8,8-tetramethyl-5,6,7,8-tetrahydro-naphthalen-2-yl-carbonyloxy)-nicotinic acid
and pharmaceutically usable salts thereof.

**14.** The compounds of claims 1-13 for use as medicaments.

**15.** A pharmaceutical preparation containing a compound of claims 1-13 and usual pharmaceutical carriers and adjuvants.

**16.** The use of a compound of claims 1-13 as an active substance in the production of medicaments for the therapy and prophylaxis of light- and age-caused damage to the skin; for the promotion of wound healing, for the therapy and prophylaxis of dermatological disorders which are accompanied by epithelial lesions,; as well as malignant and premalignant epithelial lesions, tumours and precancerous changes of the mucous membrane in the mouth, tongue, larynx, esophagus, bladder, cervix and colon.

**17.** A process for the manufacture of compounds of claims 1-13, **characterized by** reacting a compound of formula II

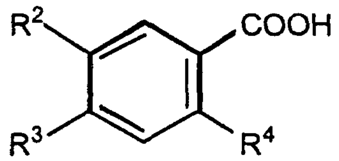

II

wherein $R^2$, $R^3$ and $R^4$ have the significance given in claim 1, with a compound of formula III

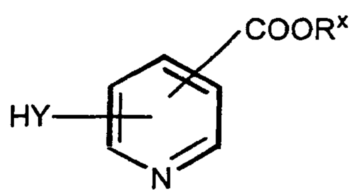

III

wherein $R^x$ represents a protecting group and Y has the significance given in claim 1.

## Revendications

**1.** Composés de formule

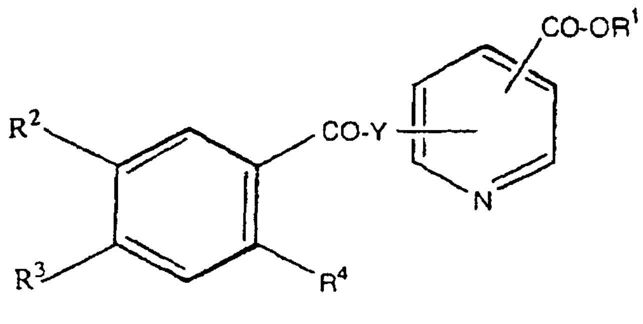

I

dans lequel
$R^1$ représente un atome d'hydrogène ou un alkyle en $C_{1-6}$ ;

$R^2$ représente un alkyle en $C_{1-6}$ ;
$R^3$ représente un alkyle en $C_{1-6}$ ; ou
$R^2$ et $R^3$ représentent ensemble $-(CR^6R^7)_n-$ ;
$R^4$ représente un alkyle en $C_{2-8}$, un alcényle en $C_{2-8}$, un alcynyle en $C_{2-8}$, $-OCH_2R^5$ ou un alcanoyle en $C_{2-8}$ ;
$R^5$ représente un alkyle en $C_{1-6}$, un alcényle en $C_{2-6}$ ou un alcynyle en $C_{2-6}$ ;
$R^6$ et $R^7$ représentent indépendamment l'un de l'autre un atome d'hydrogène ou un alkyle en $C_{1-6}$ ;
Y représente un atome d'oxygène ou de soufre ; et
n est égal à 3, 4 ou 5,
et des sels d'acides carboxyliques de formule I pharmaceutiquement acceptables.

**2.** Composés selon la revendication 1, dans lequel Y est un atome d'oxygène.

**3.** Composés selon la revendication 1, dans lequel Y est un atome de soufre.

**4.** Composés selon l'une des revendications 1 à 3, dans lequel $R^2$ et $R^3$ représentent ensemble $-(CR^6R^7)_n-$.

**5.** Composés selon la revendication 4, dans lequel $R^2$ et $R^3$ représentent ensemble un groupement $-C(CH_3)_2CH_2CH_2C(CH_3)_2-$, $-C(CH_3)_2(CH_2)_4-$ ou $-C(CH_3)_2(CH_2)_2-$.

**6.** Composés selon la revendication 5, dans lequel $R^4$ est un alkyle en $C_{2-8}$.

**7.** Composés selon la revendication 5, dans lequel $R^4$ est un alcényle en $C_{2-8}$.

**8.** Composés selon la revendication 5, dans lequel $R^4$ est un alcanoyle en $C_{2-8}$.

**9.** Composés selon la revendication 5, dans lequel $R^4$ est $-OCH_2R^5$.

**10.** Composé selon la revendication 6, l'acide nicotinique 6-(3-hexyl-5,5,8,8-tétraméthyl-5,6,7,8-tétrahydro-naphthaline-2-yl-carbonyloxy) et ses sels pharmaceutiquement acceptables.

**11.** Composés selon la revendication 6,
l'acide nicotinique 6-(3-pentyl-5,5,8,8-tétraméthyl-5,6,7,8-tétrahydro-naphthaline-2-yl-carbonyloxy),
l'acide nicotinique 6-(3-hexyl-5,5-diméthyl-6,7,8,9-tétrahydro-5H-benzocycloheptène-2-yl-carbonyloxy), et
l'acide nicotinique 6-(6-hexyl-3,3-diméthyl-indan-5-yl-carbonyloxy), et leurs sels pharmaceutiquement acceptables.

**12.** Composés selon la revendication 7, l'acide nicotinique 6-(3-(Z)-1-hexényl-5,5,8,8-tétraméthyl-5,6,7,8-tétrahydro-naphthaline-2-yl-carbonyloxy) et ses sels pharmaceutiquement acceptables.

**13.** Composés selon la revendication 9,
l'acide nicotinique 6-(3-butoxy-5,5,8,8-tétraméthyl-5,6,7,8-tétrahydro-naphthaline-2-yl-carbonyloxy) et
l'acide nicotinique 6-(3-pentoxy-5,5,8,8-tétraméthyl-5,6,7,8-tétrahydro-naphthaline-2-yl-carbonyloxy)
et leurs sels pharmaceutiquement acceptables.

**14.** Composés selon les revendications 1 à 13 pour une utilisation en tant que médicament.

**15.** Préparations pharmaceutiques contenant un composé des revendications 1 à 13 et des substances de support et des adjuvants pharmaceutiques usuels.

**16.** Utilisation d'un composé selon les revendications 1 à 13 en tant que substance active pour la fabrication de médicaments pour la thérapie et la prophylaxie de lésions cutanées provoquées par la lumière ou l'âge ; pour favoriser la cicatrisation, pour la thérapie et la prophylaxie de maladies dermatologiques accompagnées de lésions épithéliales ; ainsi que de lésions malignes et prémalignes, de tumeurs et de mutations précancéreuses des muqueuses de la bouche, de la langue, du larynx, de l'oesophage, de la vessie, du col de l'utérus et du colon.

**17.** Procédé pour fabriquer des composés selon les revendications 1 à 13, **caractérisé en ce qu'**un composé de formule II

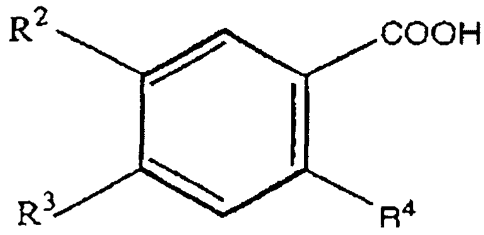

II

dans lequel $R^2$, $R^3$ et $R^4$ ont la signification indiquée dans la revendication 1, est transformé en présence d'un composé de formule III

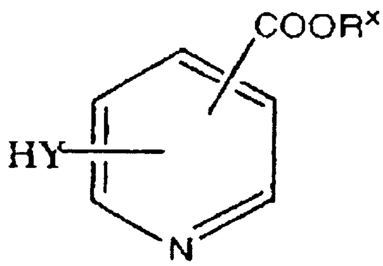

III

dans lequel $R^x$ représente un groupe de protection et Y a la signification indiquée dans la revendication 1.